**Europäisches Patentamt**

**European Patent Office**

**Office européen des brevets**

(11) Publication number: **0 118 275**
**B1**

(12) **EUROPEAN PATENT SPECIFICATION**

(45) Date of publication of patent specification: **25.01.89**

(21) Application number: **84301279.0**

(22) Date of filing: **28.02.84**

(51) Int. Cl.⁴: **C 12 M 1/20, C 12 M 1/34**

(54) Microbiological testing apparatus.

(30) Priority: **04.03.83 US 472416**

(43) Date of publication of application:
**12.09.84 Bulletin 84/37**

(45) Publication of the grant of the patent:
**25.01.89 Bulletin 89/04**

(84) Designated Contracting States:
**AT BE CH DE FR GB IT LI LU NL SE**

(56) References cited:
**EP-A-0 045 041**
**FR-A-1 221 896**
**FR-A-2 110 030**
**FR-A-2 279 847**
**US-A-4 284 725**

(73) Proprietor: **AMERICAN HOME PRODUCTS**
**CORPORATION**
**685, Third Avenue**
**New York, New York 10017 (US)**

(72) Inventor: **Godsey, James Hal**
**21 East 17th Street**
**Huntington Station New York 11746 (US)**

(74) Representative: **Chettle, Adrian John et al**
**c/o John Wyeth & Brother Limited Huntercombe**
**Lane South**
**Taplow Maidenhead Berkshire, SL6 0PH (GB)**

Courier Press, Leamington Spa, England.

## Description

This application relates to microbiological testing apparatus. More particularly it relates to improved microbiological test trays which can be read in either a manual or an automatic reader.

Different types of microbiological testing are usually carried out in trays which have a number of chambers. The chambers which are also known as test wells or cupules are prepared by the manufacturer to contain test reagents. Typically the test reagents are complex chemicals which in the presence of an active fermenting culture change colour, become cloudy or otherwise indicate that fermentation is or has taken place. The absence of an indication of such activity is a negative result which can be as significant as a positive result in the identification of unknown microorganisms or in determining the susceptibility of a microorganism for attack by an antibiotic. Such test trays may also be used to determine what microorganism is causing the illness of a patient.

Typically the test reagents are charged into the chambers in the form of an aqueous solution and any growth medium may also be charged at the same time. Usually a different combination of reagent or growth medium is charged into successive chambers so that a great number of individual reactions are performed in a physically small apparatus.

After charging the test trays are placed into a lyophilizer where the ingredients are first frozen and then the water is removed by sublimation upon the lowering of the ambient pressure to form a vacuum. It is the test tray containing the dried reagents which is sold and used for carrying out microbiological tests; many different precharged test trays are available, each with different combinations and strengths of reagent and/or growth medium.

When a test is to be performed a microorganism is inoculated into each of the test chambers with sufficient water to reconstitute the reagents. The test tray is then incubated at an appropriate temperature, usually elevated above ambient, for an extended period of time. After a predetermined period the individual chambers are examined for the presence or absence of a reaction or an indication of colour change, or a change in turbidity. While these chambers may be read visually by a technician there have been developed a number of reading machines which facilitate the reading of the trays and result in the saving of technicians' time.

A typical tray may contain 20 to 120 chambers for individual reactions. As tests have grown more complex more chambers have been needed. Also different test trays are used to determine different characteristics of the microorganisms. While trays can be read individually, each requires the use of technicians' time in the preparation, inoculation, incubation and reading of the results.

One of the standard tests is described in United States Patents 3936356 and 4056359. In this patent is described a 20 cupule test strip in which a single row of 20 cupules is arranged side by side each containing a different reagent. By reading the cupules in which reactions occur an unknown microorganism can be identified with reference to a manual of reactions.

The nature of the cupules and their use in microbiological testing is described in United States Patents 3854883, 3876378 and 4208480.

A typical automatic reader is manufactured by Dynatech Corporation, Alexandria, Va, United States of America as model Mr 600. A typical manual reader is made by Bellco Glass, Inc., Vineland, New Jersey, United States of America.

One problem with existing test trays sold for microbiological tests is that the particular reagents desired for a particular test are not always available in a single tray. Consequently several trays must be used and it is relatively easy for the associated trays to become separated or confused during incubation which may take place with other similar test trays used for a different microbiological test. The associated trays may also be confused during reading.

One alternative would be to use test trays having a very large number of individual chambers each containing a different combination of reagent or growth medium. In this way a single large test tray could be used for each microbiological test. This would however be very costly and wasteful as a large number of chambers would remain unused and machines of increased size would be required for lyophilization, incubation and reading.

The present invention seeks to overcome these disadvantages by providing an improved microbiological test tray.

According to the invention there is provided a microbiological test tray having a plurality of chambers adapted to receive reagents in solution, the reagents being lyophilized to dryness, the tray having a skirt around at least part of the periphery thereof and fastening means being provided on the skirt whereby a plurality of test trays may be joined together.

In this way the user may select a number of small test trays having, in total, the required combinations of reagents and/or growth medium and join them together to form a unitary test tray having the minimum number of unused test chambers. Considerable saving may thus be made, both by a reduction in the number of wasted test chambers and by greater utilization of lyophilization, incubation and reading equipment. The greater density of relevant test chambers is beneficial to the user when comparing results in different chambers.

Microbiological test trays are usually rectangular and made of plastic. The fastening means may conveniently be provided on two opposite sides thereof and may be integral with the skirt. Co-operating male and female fasteners may be moulded with the test tray by any convenient means and of any suitable type. In one preferred embodiment the male fastener comprises two spaced projections having oppositely facing hook portions; the female fasteners are co-operating apertures provided on the opposite side of the tray in register with the hooked projections. The hooked projections enter the corresponding apertures of a similar test tray and engage on the inner side of the skirt, the ends of the projections are preferably tapered to allow easy assembly of the trays. This arrangement provides an economical and effective means of joining together test trays.

The tray skirts may be shaped to allow stacking of similar trays for lyophilization. The skirt may for example have upper and lower abutment surfaces and have register means to locate one tray on another. The register means may be a rim extending outwardly and downwardly of the skirt and for close fitting engagement over the skirt of another test tray.

The invention further provides openings in the skirt to allow sublimed moisture to escape from the test chambers during lyophilization. The openings preferably extend along opposite sides of the test tray and substantially enhance the sublimation of moisture so reducing the time required for lyophilization.

In one preferred embodiment the fasteners are provided on two opposite sides of the test tray and the openings extend along the two other opposite sides.

A cover may be provided for the test tray, or for the uppermost of a stack of test trays, and the tray skirt may include means to engage and retain the cover over the test chamber. The engagement means may be a lug of the cover, which is usually made of transparent plastic material, and an aperture of the skirt. Corresponding lugs and apertures are preferably provided on opposite sides of the cover and tray.

The test tray may comprise an assembly of a base including the skirt and fasteners and a removable test card having test chambers formed therein. The test card preferably comprises two moulded plastic strips joined together to form a plurality of individual chambers to contain a dehydrated reagent. The chambers are preferably in two rows of ten.

In a preferred embodiment means are provided on the base to hold the test card in a particular orientation. Register means, such as upstanding walls, may be provided to locate the card and fastening means to resiliently hold the card relative to the base are desirable.

A single transparent template may be provided for an assembly of two or more test trays, the template having one or more opaque areas to mask unused test chambers. This arrangement has the advantage that only one template is required for the complete tray assembly instead of the several templates previously provided. The opaque areas of the template ensure that the viewers attention is directed only to those test chambers which are relevant. Means to locate the template relative to the assembly may also be provided in any convenient manner.

Further aspects of the invention will be disclosed in the following description of a preferred embodiment shown, by way of example only in the accompanying drawings, in which:—

Figure 1 is a plan view of a 120 chamber microbiological test tray;

Figure 2 is a rear elevation of the tray of Figure 1;

Figure 3 is a side elevation of the tray of Figure 1 taken partly in section along line 3-3 of Figure 1;

Figure 4 is a perspective view from above of a cover for the tray of Figure 1;

Figure 5 is a partial elevation of a plurality of the trays of Figure 1 stacked one on top of the other;

Figure 6 is a plan view of a 20 cupule microbiological test card; combined with its holder;

Figure 7 is a side elevation of the combination of Figure 6;

Figure 8 is a rear elevation of the combination of Figure 6;

Figure 9 is a transverse section taken generally along line 9-9 of Figure 6;

Figure 10 is an exploded plan view showing separately the components of the combination of Figure 6;

Figure 11 is a partial transverse section taken generally along line 11-11 of Figure 6;

Figure 12 is a plan view of a 40 chamber microbiological test tray;

Figure 13 is a rear elevation of the tray of Figure 12.

Figure 14 is a side elevation of the tray of Figure 12 taken partly in section along lines 14-14 of Figure 12;

Figure 15 is a plan view of the trays of Figures 1, 6 and 12 joined together;

Figure 16 is a plan view of a template; and

Figure 17 is a plan view of an alternative template.

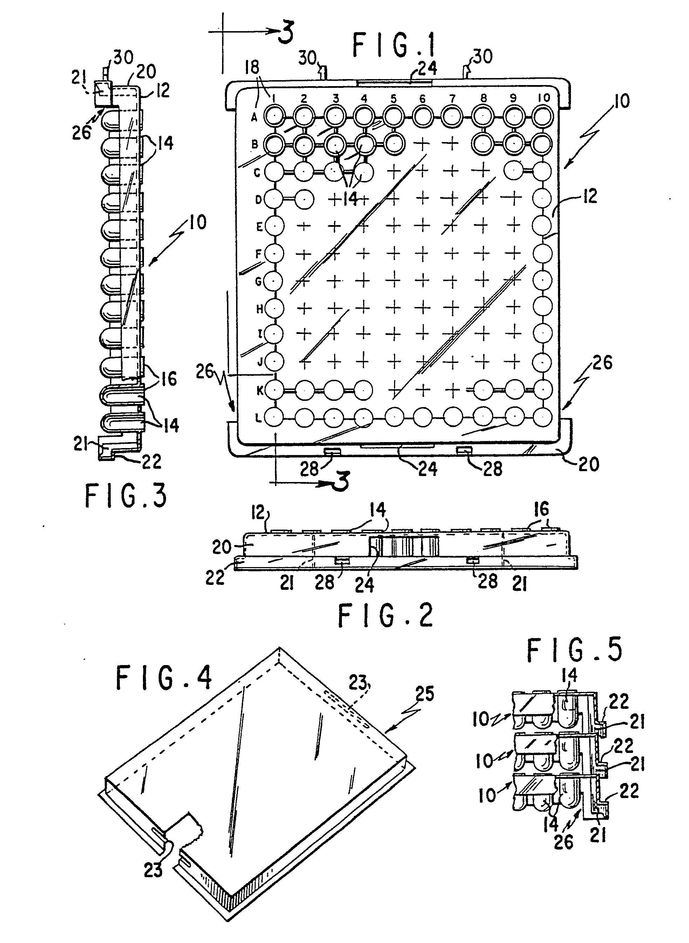

As shown in Figures 1, 2 and 3 a biological test tray 10 has supported in its upper surface 12 a plurality of chambers 14 the upper rims 16 of which extend slightly above the upper surface 12. Indicia 18 are applied at the end of each row and column in order to specify a particular chamber. Surrounding the tray is a skirt 20 which extends downwardly from the upper surface and has a shoulder 22 and lugs 21 formed in it. The shoulder, lug and skirt combination permit the stacking of the trays one on top of the other during processing and locate successive trays as illustrated further in Figure 5.

A cutaway portion 26 is provided at opposite sides of the skirt 20 in order to permit vapor flow during lyophilization. The size of the opening is not critical provided it is above the minimum necessary for effective flow, but it has been found that the openings increase the number of test trays which may undergo lyophilization at any one time as well as reducing the lyophilization time due to improved vapour flow. An opening 24 is provided on opposite sides of the tray to co-operate with a hooked male snap-on fastener 23 of a cover 25 (Figure 4) to be placed over the tray and skirt. The opening 24 shown is approximately equal in height to the diameter of the chambers and four times as long as the diameter of the chambers.

The test trays are preferably used in conjunction with an automatic reading machine. For some tests it is desirable to have more than the 120 chambers shown in Figure 1 with the same or different ingredients. For convenience in processing such as simultaneous inoculation, incubation and reading it has been found desirable to join two trays together for this purpose. Openings 28 are provided to receive a male snap fastener attached to a second type of tray as will be described below. If desired, male fasteners 30 may also be attached to the tray skirt opposite the openings 28 to provide for connection of more than two trays of the type shown in Figure 1. The design of the fasteners and openings is not critical, and any well know combination of stud and opening may be used for the purpose. The trays are preferably made of a slightly flexible plastic which imparts sufficient flexibility to the male fasteners 30 to be engaged and disengaged in the openings 28.

In United States Patent 4056359 assigned to Analytab Products Inc., there is described apparatus for identifying bacteria. In Figure 1 of the '359 patent there is shown a typical biological test strip of twenty individual reaction chambers. The test strip is known in the art as the "API 20".

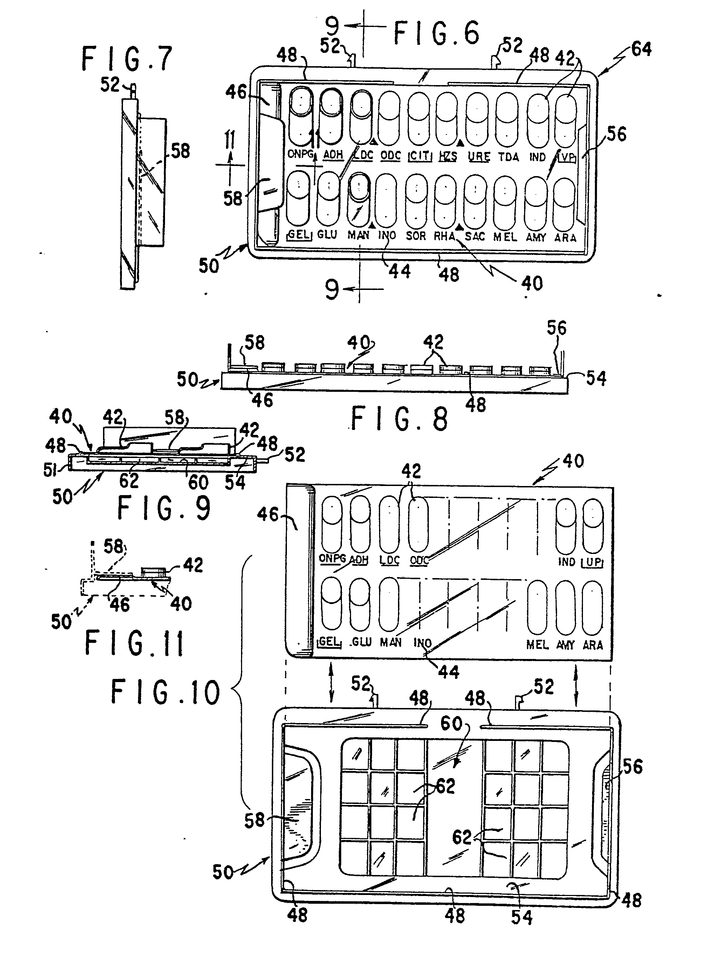

Figures 6 to 11 show a version of the test strip which has been modified into the form of a card 40. The card is formed from two moulded plastic strips joined together to form twenty individual chambers. If desired, indicia, 44 may be associated with each cupule in order to identify its contents. Preferably, the indicia correspond with identification standards promulgated by the National Committee for Clinical Laboratory Standards (of the United States of America). The cupules are arranged in two rows of ten in order to co-operate and align with the ten rows of the tray shown in Figure 1.

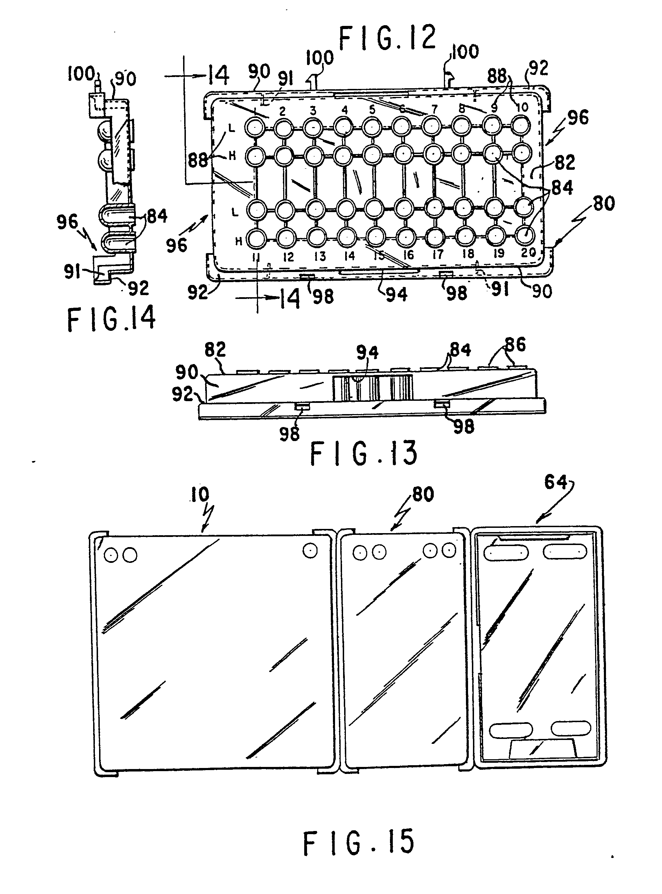

A tray 50 is provided to hold the card 40 in a combination 64 and has a skirt 51 provided with male fasteners 52 which may engage the openings 28 of the tray of Figure 1 in order to secure the card 40 and the tray 50 to the tray 10. The skirt may also have openings (not shown) corresponding to openings 28 of Figure 1 and to receive male fasteners. The tray 50 is made of a clear plastic material having a base portion 54, to which the fasteners 52 are attached and retaining arms 56, 58 which extend over the ends of the card 40 to hold it in position. Retaining arm 58 is wider than the retaining arm 56 so that the card 40 can be inserted only in one orientation, the orientation being provided by the indexing portion 46 which is a wide portion at one end only of the card 40. As is shown in Figure 11 the indexing poriton may be dished or thickened to grip the card 40 by friction. A ridge 48 is provided to locate and retain the card 40 in the tray 50. Other additional retaining devices and structural members may be added to provide rigidity and aid registry.

It has also been found desirable to provide recessed portion 60 which is divided into a series of recessed chambers 62. In use these chambers may contain water to provide humidification which is desirable during incubation. The card 40 is above the recessed portion 60 when in place in the tray 50. Openings (not shown) may be provided in the skirt 51 to provide improved vapour flow during lyophilization.

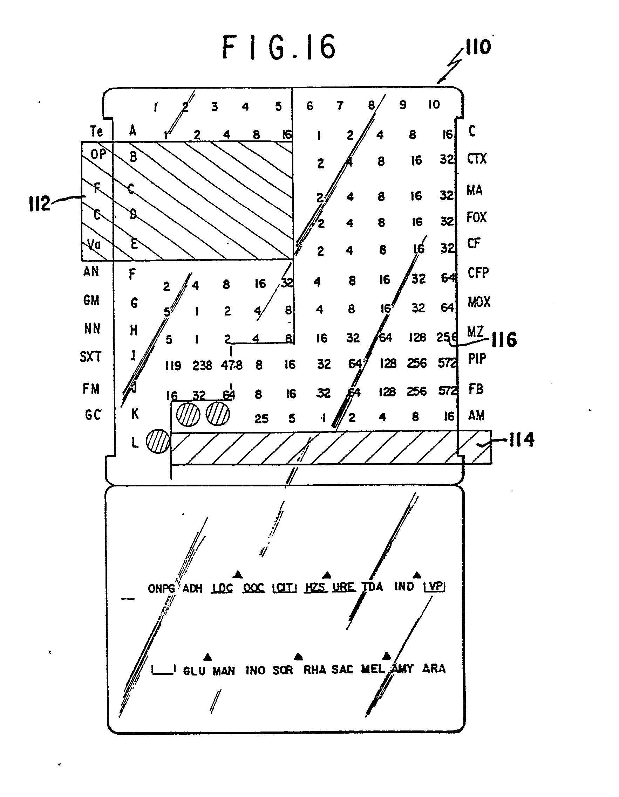

In Figures 12 to 14 is shown a further biological test tray 80 having an upper surface 82 and forty chambers 84 arranged in four rows of ten with the upper rims 86 extending slightly above the upper surface 82. Indicia 88 are preferably associated with each chamber. A skirt 90 surrounds the test tray and is attached to the upper surface 82. A shoulder 92 and lugs 91 are formed in the skirt for convenience in stacking during processing. It has been found advantageous to have a cutaway portion 96 of the skirt at opposite ends as described above with reference to cutaway portion 26 of tray 10. Openings 98 are formed on the skirt on one side and are adapted to connect with fasteners on other trays. Male fasteners 100 may be formed on the opposite side of the skirt for connection to other trays having female openings. A cover (not shown) similar to cover 25 may be fitted over the tray and skirt to cover the chambers. Openings 94 are provided to receive a snap fastener portion of the cover and hold it in place. Figure 15 shows an assembly of test trays which may be required for a particular microbiological test and which comprises one each of the test trays of Figures 1, 6 and 12.

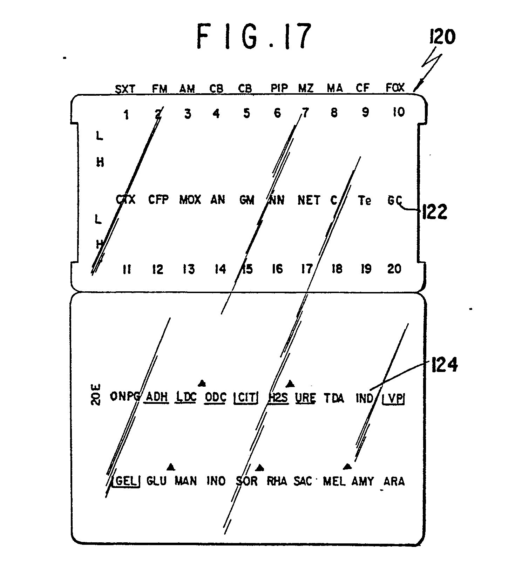

Not all of the chambers need be read for all tests performed. It is therefore advantageous to provide templates which indicate those selected chambers which are to be read in order to obtain the results for a particular test. Typical templates 110 and 120 are shown in Figures 16 and 17 and are typically a transparent plastic sheet of the same size as the combination of trays which are to be read. The areas 112 and 114 may be made opaque to exclude chambers that are not to be read in the test associated with the template 110. Indicia 116, 122, 124 may be present for ease in reading. The present invention by use of a number of trays coupled together permits the use of templates of larger size in which the selected chambers from more than one test tray may be read at the same time. It is the rigidity of the connection between the individual microbiological test trays which permits the use of precut templates with the test trays.

**Claims**

1. A microbiological test tray having a plurality of test chambers adapted to receive reagents in solution, the reagents being lyophilized to dryness, characterized thereby that the tray (10, 64, 80) has a skirt (20, 51, 90) around the periphery thereof, the skirt including an upper and lower abutment face, whereby a plurality of simular test trays may be stacked one on another in registration, and openings (26,96) in the skirt to enhance the evacuation of moisture.

2. A test tray according to Claim 1 characterised thereby that the skirt (20, 51, 90) has oppositely facing openings.

3. A test tray according to Claim 1 or Claim 2, characterised thereby that fastening means (28, 30, 52; 98, 100) are provided on said skirt whereby a plurality of test trays may be joined together.

4. A test tray according to Claim 3, characterised thereby that the tray (10, 64, 80) is substantially rectangular, said fastening means being provided on two opposite sides thereof.

5. A test tray according to Claim 3, characterised thereby that male fastening means (30, 52, 100) are provided on one side of the tray and female fastening means (28, 98) on the other side.

6. A test tray according to Claim 5, characterised thereby that said male fastening means (30, 52, 100) comprise two spaced projections having oppositely facing hook portions and said female fastening means comprise two spaced apertures (28, 98) in register with said projections.

7. A test tray according to any preceding claim, characterised thereby that said skirt (20, 51, 90) further includes means (24) to engage and retain a cover (25) for the test chambers.

8. A test tray according to any preceding claim, characterised thereby that said tray (64) comprises a base (50) including said skirt (51) and a removable test card (40) having chambers adapted to receive reagents, the base further including engagement means (56, 58) to engage and retain said test card.

9. A test tray according to Claim 8, characterised thereby that the base (50) further includes register means (56, 58) for co-operation with corresponding register means of said test card (40) to ensure said base and card are assembled in a particular orientation.

10. An assembly of two or more test trays, according to any preceding claim and connected together by said fastening means characterised thereby that a single transparent template (110, 112) is provided to cover the test chambers of said assembly, said template having opaque areas (112, 114) to mask certain test chambers from view.

**Patentansprüche**

1. Mikrobiologische Testschale mit einer Mehrzahl von Testkammern zur Aufnahme von Reagentien in Lösung, wobei die Reagentien bis zur Trockene gefriergetrocknet werden, dadurch gekennzeichnet, daß die Schale (10, 64, 80) rund um ihren Umfang eine Einfassung (20, 51, 90), die eine obere und eine untere Auflagerfläche aufweist, wodurch eine Mehrzahl von ählichen Testschalen in Ausrichtung zueinander übereinander stapelbar ist, und Öffnungen (26, 96) in der Einfassung hat, um den Abtransport von Feuchtigkeit zu verstärken.

2. Testschale nach Anspruch 1, dadurch gekennzeichnet, daß die Einfassung (20, 51, 90) entgegengesetzt gerichtete Öffnungen hat.

3. Testschale nach Anspruch 1 oder 2, dadurch gekennzeichnet, daß Befestigungsmittel (28, 30, 52; 98, 100) an der Einfassung vorgesehen sind, wodurch eine Mehrzahl von Testschalen miteinander verbindbar ist.

4. Testschale nach Anspruch 3, dadurch gekennzeichnet, daß die Schale (10, 64, 80) im wesentlichen rechteckig ist, wobei die Befestigungsmittel an zwei gegenüberliegenden Seiten hievon vorgesehen sind.

5. Testschale nach Anspruch 3, dadurch gekennzeichnet, daß männliche Befestigungsmittel (30, 52, 100) an einer Seite der Schale und weibliche Befestigungsmittel (28, 98) auf der anderen Seite vorgesehen sind.

6. Testschale nach Anspruch 5, dadurch gekennzeichnet, daß die männlichen Befestigungsmittel (30, 52, 100) zwei mit Abstand angeordnete Vorsprünge aufweisen, die entgegengesetzt gerichtete Hakenteile haben, und die weiblichen Befestigungsmittel zwei mit Abstand vorgesehene Öffnungen (28, 98) in Ausrichtung zu den Vorsprüngen aufweisen.

7. Testschale nach einem der vorhergehenden Ansprüche, dadurch gekennzeichnet, daß die Einfassung (20, 51, 90) weiters Mittel (24) für einen Eingriff mit einer Abdeckung (25) für die Testkammern und für deren Festhalten aufweist.

8. Testschale nach einem der vorhergehenden Ansprüche, dadurch gekennzeichnet, daß die Schale (64) einen die Einfassung (51) aufweisenden Unterteil (50) und eine abnehmbare Testtafel (40) mit zur Aufnahme von Reagentien geeigneten Kammern aufweist, wobei der Unterteil weiters Eingriffsmittel (56, 58) für einen Eingriff mit der Testtafel und für deren Festhalten aufweist.

9. Testschale nach Anspruch 8, dadurch gekennzeichnet, daß der Unterteil (50) weiters Ausrichtmittel (56, 58) zum Zusammenarbeiten mit entsprechenden Ausrichtmitteln der Testtafel (40) aufweist, um einen Zusammenbau von Unterteil und Tafel in einer speziellen Ausrichtung sicherzustellen.

10. Anordnung mit zwei oder mehreren, miteinander durch die Befestigungsmittel verbundenen Testschalen nach einem der virhergehenden Ansprüche, dadurch gekennzeichnet, daß eine einzige transparente Matrize (110, 112) zur Abdeckung der Testkammern der Anordnung vorgesehen ist, wobei die Matrize opake Bereiche (112, 114) aufweist, um bestimmte Testkammern gegenüber einer Besichtigung zu maskieren.

**Revendications**

1. Plaque pour tests microbiologiques comportant plusieurs chambres de tests propres à recevoir des réactifs en solution, les réactifs étant lyophilisés jusqu'à siccité, caractérisée en ce qu'elle (10, 64, 80) comporte une jupe (20, 51, 90) autour de sa périphérie, cette jupe comprenant une face de butée supérieure et une face de butée inférieure, de sorte que plusieurs plaques pour tests semblables peuvent être empilées en coïncidence l'une sur l'autre, et des ouvertures (26, 96) dans la jupe pour favoriser l'évacuation de l'humidité.

2. Plaque pour tests suivant la revendication 1, caractérisée en ce que la jupe (20, 51, 90) comporte des ouvertures orientées en sens opposés.

3. Plaque pour tests suivant la revendication 1 ou 2, caractérisée en ce que des moyens de fixation (28, 30, 52; 98, 100) sont prévus sur la jupe, de telle sorte que plusieurs plaques puissent être assemblées.

4. Plaque pour tests suivant la revendication 3, caractérisée en ce qu'elle (10, 64, 80) est en substance rectangulaire, les moyens de fixation étant prévus sur deux de ses côtés opposés.

5. Plaque pour tests suivant la revendication 3, caractérisée en ce que des moyens de fixation mâles (30, 52, 100) sont prévus d'un côté de la plaque et des moyens de fixation femelles (28, 98), de l'autre côté.

6. Plaque pour tests suivant la revendication 5, caractérisée en ce que les moyens de fixation mâles (30, 52, 100) comprennent deux saillies espacées comportant des crochets orientés en sens opposés et les moyens de fixation femelles (28, 98) comprennent deux ouvertures espacées en coïncidence avec les saillies.

7. Plaque pour tests suivant l'une quelconque des revendications précédentes, caractérisée en ce que la jupe (20, 51, 90) comprend, en outre, des moyens (24) destinés à engager et à retenir un couvercle (25) pour les chambres de tests.

8. Plaque pour tests suivant l'une quelconque des revendications précédentes, caractérisée en ce qu'elle (64) comprend une base (50) comprenant la jupe (51) et une carte de tests amovible (40) comportant des chambres destinées à recevoir des réactifs, la base comprenant, en outre, des moyens d'engagement (56, 58) destinés en engager et à retenir la carte de tests.

9. Plaque pour tests suivant la revendication 8, caractérisée en ce que la base (50) comprend, en outre, des moyens de repérage (56, 58) destinés à coopérer avec des moyens de repérage correspondants de la carte de tests (40) pour assurer que la base et la carte soient assemblées dans une orientation particulière.

10. Ensemble de deux ou de plusieurs plaques pour tests suivant l'une quelconque des revendications précédentes, reliées l'une à l'autre par les moyens de fixation, caractérisé en ce qu'un seul gabarit transparent (110, 112) est prévu pour couvrir les chambres de tests de l'ensemble, le gabarit comportant des zones opaques (112, 114) destinées à masquer certaines chambres de tests.

FIG.1

FIG.3

FIG.2

FIG.4

FIG.5

FIG.7

FIG.6

52
58

48    52              52  48 42    64
46                                        56
ONPG ADH LDC ODC CIT HZS URE TDA IND VP
11
58
GEL GLU MAN INO SOR RHA SAC MEL AMY ARA
50
9    44    48    40

58    40    42                56
50                                        54
46                    48

FIG.8

40  42  58          42  48
48                          52
51
50  62    60  54

FIG.9

58                    42
50    46          40

FIG.11

FIG.10

42              40
46
ONPG ADH LDC ODC              IND VP
GEL GLU MAN INO          MEL AMY ARA
44

52    48  48    52
60
56
62
58
62
50
48    48    54    48

FIG.12

FIG.14

FIG.13

FIG.15

# FIG.16

FIG.16

# F I G . 17